(19) Europäisches Patentamt
European Patent Office
Office européen des brevets

(11) Publication number: **0 380 316**
**A2**

# (12) EUROPEAN PATENT APPLICATION

(21) Application number: **90300731.8**

(51) Int. Cl.[5]: **C07C 51/00, C07C 59/64**

(22) Date of filing: **24.01.90**

(30) Priority: **25.01.89 US 300015**

(43) Date of publication of application:
**01.08.90 Bulletin 90/31**

(84) Designated Contracting States:
**AT BE CH DE DK ES FR GB GR IT LI LU NL SE**

(71) Applicant: **SYNTEX PHARMACEUTICALS INTERNATIONAL LIMITED**
**Rosebank Building, Bermudiana Road**
**Hamilton(BM)**

(72) Inventor: **Prince, Anthony**
**335 Cypress Point Drive**
**Mountain View, California 94043(US)**
Inventor: **Schloemer, George C.**
**1278 Fox Hill Drive**
**Longmont, CC 80501(US)**

(74) Representative: **Armitage, Ian Michael et al**
**MEWBURN ELLIS 2 Cursitor Street**
**London EC4A 1BQ(GB)**

## (54) Preparation of alpha-methylareneacetic acids.

(57) A process for the preparation of α-methylarene-acetic acids includes treating suitable ketals in an aqueous acidic medium to cause rearrangement of the ketal to an ester of the corresponding α-methylareneacetic acid, and hydrolyzing the thus-formed ester to yield the α-methylareneacetic acid.

EP 0 380 316 A2

# PREPARATION OF α-METHYLARENEACETIC ACIDS

This invention relates to a process for the preparation of α-methylareneacetic acids, both racemic and optically active. In particular, it relates to a process for preparing such acids by means of the rearrangement of suitable ketals in an aqueous acidic medium, then hydrolyzing the ester obtained by the rearrangement.

## Background to the Invention

Numerous α-methylareneacetic acids (2-arylpropionic acids) have been described, developed and found to be useful as pharmaceutical agents exhibiting anti-inflammatory, analgesic and anti-pyretic activity. For example, U.S. Patent No. 3,385,386 describes certain α-methylbenzeneacetic acids useful for their anti-inflammatory activity. Particularly noteworthy of the compounds described therein is α-methyl-4-(2-methylpropyl)benzeneacetic acid, known generically as ibuprofen. U.S. Patent No. 3,600,437 describes α-methyl-3-phenoxy- and α-methyl-3-phenylthiobenzeneacetic acids, among other related compounds. Particularly noteworthy therein is the compound α-methyl-3-phenoxybenzeneacetic acid, known generically as fenoprofen. U.S. Patent No. 3,624,142 describes (fluoro-substituted biphenyl)alkanoic acids, among which is 4′-fluoro-α-methyl-1,1′-biphenyl-4-acetic acid. U.S. Patent No. 3,755,427 describes additional (fluoro-substituted biphenyl)alkanoic acids, among which is 2-fluoro-α-methyl-1,1′-biphenyl-4-acetic acid, known as flurbiprofen. U.S. Patent No. 3,904,682 describes the compound 6-methoxy-α-methyl-2-naphthaleneacetic acid, the S-isomer of which is known generically as naproxen and is a potent anti-inflammatory compound. Related compounds are described in Belgian Patent No. 747,812. U.S. Patent No. 3,912,748 describes 5- and 6-benzoxazolealkanoic acids possessing anti-inflammatory, anti-pyretic and analgesic activity. Notable among those compounds is 2-(4-chlorophenyl)-α-methyl-5-benzoxazoleacetic acid, known generically as benoxaprofen. Thus, it can be seen that a tremendous variety of useful α-methylareneacetic acids are known.

Other known, useful α-methylareneacetic acids are exemplified by 6-chloro-α-methyl-9H-carbazole-2-acetic acid (carprofen), α-methyl-9H-fluorene-2-acetic acid (cicloprofen), 3-chloro-α-methyl-4-(2-thienylcarbonyl)-benzeneacetic acid (cliprofen), α-methyl-3-phenyl-7-benzofuranacetic acid (furaprofen), 4-(1,3-dihydro-1-oxo-2H-isoindol-2-yl)benzeneacetic acid (indoprofen), 3-benzoyl-α-methylbenzeneacetic acid (ketoprofen), 3-chloro-4-(2,5-dihydro-1H-pyrrol-1-yl)benzeneacetic acid (pirprofen), α-methyl-4-(2-thienylcarbonyl)-benzeneacetic acid (suprofen) and compounds related thereto.

Each of these α-methylareneacetic acids has an asymmetric centre, the carbon atom α to the carboxyl group, and exists therefore in the form of two stereoisomers. Often a decidedly higher biological activity is associated with one of the two isomers. A particularly evident example is given by 6-methoxy-α-methyl-2-naphthaleneacetic acid, whose S-isomer (naproxen) shows pharmacological properties definitely better than those of the R-isomer and of the racemic mixture. For this reason, it is useful that a stereoselective process be available, leading to the formation of the desired optical isomer of an α-methylareneacetic acid in a substantially pure form.

In addition to those α-methylareneacetic acids which are themselves useful as pharmaceutical agents, such as have been described above, certain other α-methylareneacetic acids are useful as precursors to the pharmaceutically useful α-methylareneacetic acids. Notable among these are the precursors to (RS)-6-methoxy-α-methyl-2-naphthaleneacetic acid, and its active isomer, (S)-6-methoxy-α-methyl-2-naphthaleneacetic acid (naproxen). These precursors include 6-hydroxy-α-methyl-2-naphthaleneacetic acid, the 5-halo-6-methoxy-α-methyl-2-naphthaleneacetic acids (especially 5-bromo-6-methoxy-α-methyl-2-naphthaleneacetic acid), and their S-isomers.

Within the past ten years, substantial efforts have been devoted to the preparation of α-methylareneacetic acids, both racemic and optically active, by the rearrangement of ketals of 1-aryl-2-halo-1-propanones.

For example, European Published Application No. 0 035 305 discloses the preparation of racemic cyclic and acyclic ketals of 2-halo-1-(6-methoxy-2-naphthyl)-1-propanone and 2-halo-1-(5-halo-6-methoxy-2-naphthyl)-1-propanone and their rearrangement with Lewis acids to yield esters of 6-methoxy-α-methyl-2-naphthaleneacetic acid and 5-halo-6-methoxy-α-methyl-2-naphthaleneacetic acid. European Published Application No. 0 034 871 discloses the preparation of similar ketals of 1-aryl-1-alkanones other than those described in EP 0 035 305 above.

European Published Application Nos. 0048136 and 0064394 disclose the rearrangement of ketals of an α-hydroxyalkylaryl ketones which is effected by activating the α-hydroxy moiety with an esterifying agent,

specifically an sulfonic acid ester, to form the corresponding alkylaryl ketal ester substrate.

Rearrangement of haloketones by silver (I) salts is disclosed in British Published Application No. 2 042 543.

European Published Application No. 0 081 993 discloses the preparation of optically active ketones of the formula

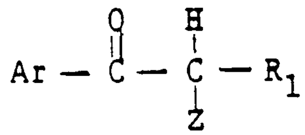

in which Ar is aryl, $R_1$ is alkyl or cycloalkyl, and Z is a leaving group, including halogen and sulfonic esters; and their ketalization to cyclic and acyclic ketals and rearrangement to yield optically active $\alpha$-alkylareneacetic acids.

European Published Application No. 0 101 124 discloses the preparation of ketals of 1-aryl-2-halo-1-alkanones, and their rearrangement and hydrolysis to the corresponding $\alpha$-alkylareneacetic acids in neutral or slightly alkaline conditions in the presence of a polar protic medium.

European Published Applications Nos. 0 151 817 and 0 153 701 disclose the rearrangement of similar ketals in neutral or slightly alkaline conditions in the presence of a dipolar aprotic diluent and of a protic substance having a high dielectric constant, and the subsequent hydrolysis of the thus-obtained ester.

U.S. Patent No. 4,697,036 discloses an enantioselective process for the preparation of optically active $\alpha$-alkylareneacetic acids by the preparation of tartaric acid ketals of 1-aryl-1-alkanones, stereoselective halogenation on the 2-carbon of the alkanones, and rearrangement of the thus-formed haloketals to esters of $\alpha$-alkylareneacetic acids. The haloketals are of the formula

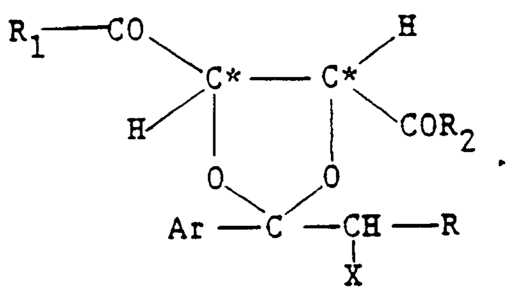

(A)

The rearrangement may be performed with Lewis acid, in alkaline solution, or by other previously-known methods; or it may be performed by treatment of the ketals with aqueous acid. The patent states, with regard to this aqueous acidic rearrangement:

> The enantioselective process object of the present invention essentially consists in rearranging a ketal of formula A in which X is a chlorine, bromine, or iodine atom, in aqueous medium at an acid pH, at a temperature comprised between room temperature and 100 °C. The above mentioned rearrangement conditions are particularly unexpected and surprising in that it is well known that treatment of a ketal with water under acidic conditions is a general method to convert ketals into the corresponding ketones and the alcohol or diol. Accordingly, the previously known alpha-haloalkylaryl ketals, under the above reaction conditions, undergo a fast hydrolysis providing the corresponding alpha haloalkyl-arylketone and alcohol or diol.
>
> On the contrary, the ketals of formula A object of the present invention, when treated in aqueous acid medium, provide in high yield the corresponding alpha-arylalkanoic acids, ketones being present, if any, in negligeable amounts.
>
> * * *
>
> Such a process is possible thanks to the unexpected characteristics of the ketals of formula A shown both in the alpha halogenation step and in the aqueous rearrangement step.

J.Org.Chem., **1987**, 52, 3018-3027, discusses the use of tartaric acid as a chiral auxiliary in the preparation of optically active $\alpha$-alkylareneacetic acids, and includes a discussion of the preparation of

optically active haloketones and a substantial list of references to prior work on ketal rearrangement.

European Published Application No. 0 210 589 discloses the preparation and rearrangement of optically active ketals similar to those disclosed in US 4,697,036 above, except that the ketals do not contain more than one carboxy group and the only rearrangement methods explicitly disclosed are Lewis acid rearrangement and rearrangement under neutral or slightly alkaline conditions.

The disclosures of each of the documents referred to in this "Background to the Invention" and elsewhere in the specification of this application are incorporated herein by reference.

This invention describes a process for the preparation of an α-methylareneacetic acid, which process comprises

(a) treating a ketal of a compound of the formula

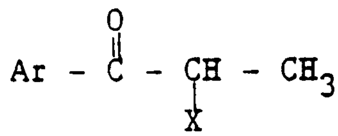

wherein Ar is an aryl group; X is halogen or a group of the formula

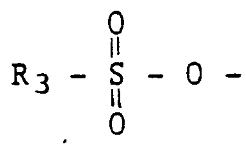

wherein $R_3$ is alkyl, cycloalkyl, alkenyl, alkynyl, aryl or arylalkyl, in an aqueous acidic medium to cause rearrangement of the ketal to an ester of the corresponding α-methylareneacetic acid; and

(b) hydrolyzing the thus-formed ester to yield the α-methylareneacetic acid, with the proviso that if the ketal is a ketal of a 1-aryl-2-halo-1-propanone and is cyclic, it contains not more than one carboxy group.

In particular, the process comprises the reaction represented schematically:

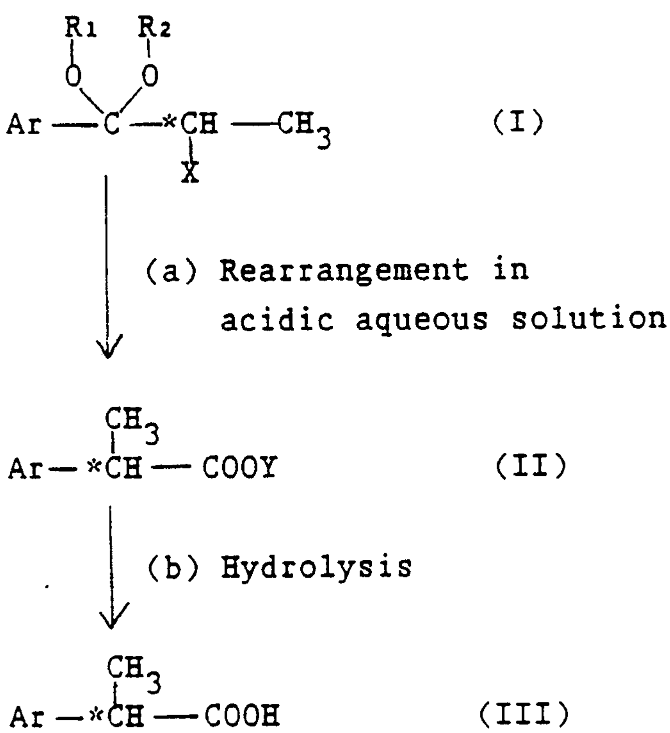


(I)

(II)

(III)

in which

Ar is aryl;

$R_1$ and $R_2$ are each independently alkyl, or $R_1$ and $R_2$ together are alkylene;
X is halogen or a group of formula

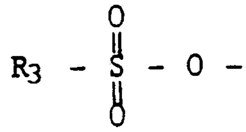

wherein $R_3$ is alkyl, cycloalkyl, alkenyl, alkynyl, aryl or arylalkyl; and
Y is H or a group supplying H by hydrolysis, the particular Y depending on the reaction conditions in rearrangement step (a).

The process of this invention is stereospecific; that is, if the ketal (1) is asymmetric at the carbon atom to which the halogen atom or the sulfonyloxy group is bonded, the resulting α-methylareneacetic acid (III) is asymmetric in the opposite sense at the α carbon, the asymmetric centres being indicated by asterisks.

If another α-methylareneacetic acid is desired, rather than the direct acid product of rearrangement and hydrolysis of the starting ketal, the process also includes a conversion step (c), in which the compound of formula II formed in rearrangement step (a) is converted to the corresponding compound of formula II with the desired arene group or the acid formed in hydrolysis step (b) is converted to the desired acid.

If a mixture of isomers of ketal (I) has been employed as the starting material but a single isomer, such as the S-isomer, of the α-methylareneacetic acid is desired, rather than the mixture of isomers prepared in hydrolysis step (b), the process also includes a resolution step (d), in which the compound of formula II is resolved to yield the desired isomer or the acid formed in hydrolysis step (b) is resolved to yield the desired isomer.

If both conversion step (c) and resolution step (d) are desired to be performed, they may be performed in either order.

If a salt of the α-methylareneacetic acid is desired, rather than the acid itself, the process also includes a salt formation step (e), in which the acid formed in hydrolysis step (b), optionally after conversion and/or resolution, is neutralized to the desired salt.

## DETAILED DESCRIPTION OF THE INVENTION

### Definitions

Unless otherwise stated, the following terms used in the specification and claims have the meanings given below:

"Alkyl", denoted generally by R, e.g. by $R_1$ or $R_2$, refers to straight or branched chain aliphatic groups having 1-12 carbon atoms or aliphatic groups having 3-12 carbon atoms and containing at least one cyclic aliphatic group (cycloalkyl groups). Those alkyl groups having 1-8 carbon atoms, and especially those having 1-4 carbon atoms, are presently preferred. The cycloalkyl groups having 3-8 carbon atoms are presently preferred. Alkyl groups include those exemplified by methyl, ethyl, cyclopropyl, cyclopropylmethyl, sec-butyl, heptyl, and dodecyl. All of the above can either be unsubstituted or substituted with one or more (preferably up to three) non-interfering substituents, e.g. halogen; $C_1$-$C_4$ alkoxy; $C_1$-$C_4$ acyloxy; hydroxy; formyl; alkylenedioxy; benzyloxy; phenyl or benzyl, each optionally substituted with from 1 to 3 substituents selected from halogen, $C_1$-$C_4$ alkyl, and $C_1$-$C_4$ alkoxy; or a carboxy group (as hereinafter defined). The term "non-interfering" characterizes the substituents as not adversely affecting any reactions to be performed in accordance with the process of this invention. If more than one alkyl group is present in a given molecule, each may be independently selected from "alkyl" unless otherwise stated. Preferred alkyl groups are $C_1$-$C_4$ alkyl, and particularly preferred are methyl and ethyl.

"Alkenyl" refers to a branched or unbranched saturated monovalent hydrocarbon chain having 2-8 carbon atoms and containing a double bond, as exemplified by vinyl, allyl, methallyl, butenyl, pentenyl, hexenyl, heptenyl, octenyl, and isomeric forms thereof.

"Alkynyl" refers to a branched or unbranched saturated monovalent hydrocarbon chain having 2-8 carbon atoms and containing a triple bond, as exemplified by ethynyl, propynyl, butynyl, pentynyl, hexynyl, heptynyl, octynyl and isomeric forms thereof.

"Alkylene" refers to a bivalent straight chain hydrocarbon radical having 2-4 carbon atoms derived from a straight chain alkane or alkene of 2-4 carbon atoms by removal of a hydrogen atom from each terminal carbon atom. The term 2-4 carbon atoms refers to the number of carbon atoms in the alkylene radical, not counting any substituents. The alkylene radical may be substituted with one or more (preferably up to four) non-interfering substituents, e.g. those selected from $C_1$-$C_{10}$ alkyl, optionally substituted with from 1 to 4 substituents selected from halogen, $C_1$-$C_4$ alkoxy, hydroxy, formyl, alkylenedioxy, and benzyloxy; phenyl or benzyl, each optionally substituted with from 1 to 3 substituents selected from halogen, $C_1$-$C_4$ alkyl, and $C_1$-$C_4$ alkoxy; or not more than one of the substituents may be a carboxy group (as hereinafter defined). Exemplary alkylene groups include
$-(CH_2)_2-$, $-CH(CH_3)-CH(CH_3)-$, $-(CH_2)_3-$, $-CH_2-C(CH_3)_2-CH_2-$, $-(CH_2)_4-$, $-CH_2-CH=CH-CH_2-$, etc.
Preferred alkylene groups are those with three carbon atoms in the alkylene radical; and particularly preferred are $-(CH_2)_3-$ and $-CH_2-C(CH_3)_2-CH_2-$.

"Aryl", denoted by Ar, includes monocyclic or condensed carbocyclic and heterocyclic aromatic groups having from 6 to 20 carbon atoms and up to three hetero atoms, such as phenyl, naphthyl, carbazolyl, and benzoxazolyl. These groups may be substituted with one or more, preferably up to three, non-interfering substituents, e.g. those selected from $C_1$-$C_4$ alkyl; halogen; $C_1$-$C_4$ alkoxy; hydroxy; formyl; alkylenedioxy; phenyl, phenoxy, benzyl or benzoyl, each optionally substituted with from 1 to 3 substituents selected from halogen, $C_1$-$C_4$ alkyl, $C_1$-$C_4$ alkoxy, and $NR_4R_5$ (where $R_4$ and $R_5$ are independently selected from H, $C_1$-$C_4$ alkyl, $C_3$-$C_6$ cycloalkyl, $-(CH_2)_n-OH$ (where n is 2-4), or $R_4$ and $R_5$ taken together are $-(CH_2)_m-$ (where m is 4-5), or $-(CH_2)_2-W-(CH_2)_2-$ where W is O, NH, or N-($C_1$-$C_4$ alkyl). In particular, the aryl groups comprehended include those of the α-methylareneacetic acids (2-arylpropionic acids) described in the "Background to the Invention" section of this application. Specific examples of aryl groups include 4-isobutylphenyl, 3-phenoxyphenyl, 2-fluoro-4-biphenylyl, 4′-fluoro-4-biphenylyl, 4-(2-thienylcarbonyl)phenyl, 6-methoxy-2-naphthyl, 5-chloro-6-methoxy-2-naphthyl, 5-bromo-6-methoxy-2-naphthyl, 6-hydroxy-2-naphthyl, and 5-bromo-6-hydroxy-2-naphthyl. Preferred aryl groups are 6-methoxy-2-naphthyl, 5-chloro-6-methoxy-2-naphthyl, 5-bromo-6-methoxy-2-naphthyl, 6-hydroxy-2-naphthyl, and 5-bromo-6-hydroxy-2-naphthyl; and a particularly preferred aryl group is 6-methoxy-2-naphthyl.

"Arylalkyl" refers to an alkyl group as defined above which is substituted with an aryl group as defined above.

"Carboxy group" refers to a group of the formula COZ where Z is OH, $O^-M^+$ (where M is the cation of an alkali metal), $OR_3$ (where $R_3$ is alkyl), or $NR_4R_5$ (where $R_4$ and $R_5$ are independently selected from $C_1$-$C_4$ alkyl, $C_3$-$C_6$ cycloalkyl, $-(CH_2)_n-OH$ (where n is 2-4), or $R_4$ and $R_5$ taken together are $-(CH_2)_m-$ (where m is 4-5), or $-(CH_2)_2-W-(CH_2)_2-$ where W is O, NH, or N-($C_1$-$C_4$ alkyl).

"Halogen", denoted by R, refers to chlorine, bromine, and iodine; with bromine and chlorine being presently preferred, and bromine being most preferred.

## Starting Materials

The ketals which are the starting materials for the rearrangement step of the process of this invention may be prepared by methods conventional in the art of ketal synthesis, such as are exemplified in the references cited in the "Background of the Invention" section of this application. Thus, for example, the syntheses of racemic α-haloketals are discussed in EP 0 035 305 and EP 0 034 871; the synthesis of racemic α-sulfonyloxyketals are discussed in EP 0048136 and EP 0064394; the syntheses of optically active ketals are discussed in EP 0 081 993 and US 4,697,036; all previously referred to.

In particular, the α-haloketals may be prepared by ketalization of racemic or optically active haloketones. The α-sulfonyloxyketals may be prepared from the α-haloketones via the α-hydroxyketals. The syntheses of suitable optically active haloketones are discussed, for example, in EP 0 081 993 and the J.Org.Chem. article, both previously referred to, and in European Published Application No. 0 067,698; and ketalization techniques are well known, as exemplified by the previously referred to patents and article. If a racemic ketone is used, a stereospecific ketalization is not required.

A person of ordinary skill in the art should thus, having regard to this disclosure and his own skill, be able to prepare the starting ketals of this invention without undue experimentation.

## Ketal Rearrangement and Hydrolysis

We have discovered, surprisingly in view of the statement set forth in U.S. Patent No. 4,697,036, that it

is possible to rearrange a ketal of a compound of the formula

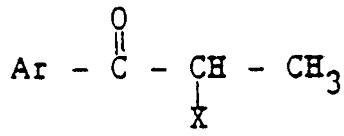

wherein Ar is an aryl group; X is halogen or a group of the formula

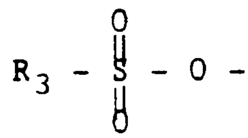

wherein $R_3$ is alkyl, cycloalkyl, alkenyl, alkynyl, aryl or arylalkyl (with the proviso that if the ketal is a ketal of a 1-aryl-2-halo-1-propanone and is cyclic, it contains not more than one carboxy group), in an aqueous acidic medium to cause rearrangement of the ketal to an ester of the corresponding α-methylareneacetic acid without substantial decomposition to the ketones.

The inventors of US 4,697,036 are true in the field of ketal rearrangement as they have been named as inventors in more than 15 European patent applications and also authored numerous articles relating to the subject matter of ketal rearrangement.

In presently preferred embodiments of this invention, there are prepared (RS)-6-methoxy-α-methyl-2-naphthaleneacetic acid and (5)-6-methoxy-α-methyl-2-naphthaleneacetic acid (naproxen); either directly by rearrangement and hydrolysis of ketals of (RS)- and (S)-2-halo-1-(6-methoxy-2-naphthyl)-1-propanone, or (RS)-and (S)-2-sulfonyloxy-1-(6-methoxy-2-naphthyl)-1-propanone, or by rearrangement and hydrolysis of 5-chloro-6-methoxy- 2-naphthyl, 5-bromo-6-methoxy-2-naphthyl, 6-hydroxy-2-naphthyl, and 5-bromo-6-hydroxy-2-naphthyl halo- or sulfonyloxy-ketal precursors followed by conversion and/or resolution to yield the desired acids. These preferred embodiments include the preparation of salts of naproxen, such as naproxen sodium.

The process of this invention is stereospecific; that is, if the ketal (I) is asymmetric at the carbon atom to which the halogen atom or the sulf onyloxy group is bonded, the resulting α-methylareneacetic acid (III) is asymmetric in the opposite sense at the α carbon, the asymmetric centres being indicated by asterisks in the formulae shown on page 7. That is, the rearrangement step (a) proceeds with inversion of configuration; but, because of the Sequence Rules used in assigning absolute configurations, an (S)-ketal gives an (S)-acid and vice versa. Ketal (I) may contain additional asymmetric centre(s), e.g. asymmetric centre(s) located on the ketal ring when the ketal is derived from an asymmetric diol such as propylene glycol, an asymmetric butanediol, and the like. These additional asymmetric centre(s) do not substantially affect the stereoselectivity of the process of this invention.

As previously described, the process of this invention comprises a rearrangement step (a), in which a ketal of a compound of the formula

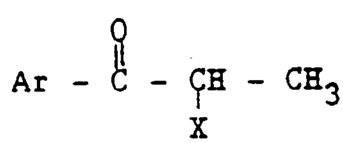

wherein Ar is an aryl group; X is halogen or a group of the formula

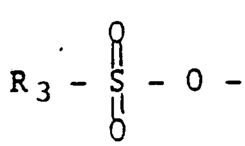

wherein $R_3$ is alkyl, cycloalkyl, alkenyl, alkynyl, aryl or arylalkyl (with the proviso that if the ketal is a ketal of a 1-aryl-2-halo-1-propanone and is cyclic, it contains not more than one carboxy group), is rearranged to an ester of the corresponding α-methylareneacetic acid, and a hydrolysis step (b), in which the ester formed in rearrangement step (a) is hydrolyzed to the acid. If another α-methylareneacetic acid is desired, rather than the direct acid product of rearrangement and hydrolysis of the starting ketal, the process also includes a conversion step (c), in which the compound of formula II formed in rearrangement step (a) is converted to the corresponding compound of formula II with the desired arene group or the acid formed in hydrolysis step (b) is converted to the desired acid. If a mixture of isomers of ketal (I) has been employed as the starting material but a single isomer, such as the S-isomer, of the α-methylareneacetic acid is desired, rather than the mixture of isomers prepared in hydrolysis step (b), the process also includes a resolution step (d), in which the compound of formula II is resolved to yield the desired isomer or the acid formed in hydrolysis step (b) is resolved to yield the desired isomer. If both conversion step (c) and resolution step (d) are desired to be performed, they may be performed in either order. If a salt of the α-methylareneacetic acid is desired, rather than the acid itself, the process also includes a salt formation step (e), in which the acid formed in hydrolysis step (b), optionally after conversion and/or resolution, is neutralized to the desired salt.

Rearrangement step (a) is carried out by treatment of the ketal (I) in an aqueous acidic medium. Use of this medium offers advantages over prior art rearrangements of these ketals, as it avoids the use of the heavy metals or transition metals associated with the Lewis acid catalysts of the prior art.

The aqueous acidic medium suitable for the process of this invention has a pH between 1 and 6, preferably between 1.5 and 5. The pH of the medium is preferably maintained within the desired range by the use of a weak acid and/or a buffer system. Suitable acidic aqueous media include aqueous solutions of lower alkanoic acids, e.g. $C_1$-$C_6$ alkanoic acids such as acetic or propanoic acids, comprising between 10% and 90% by volume of water and between 90% and 10% of one or more lower alkanoic acids. The medium may be buffered by the addition of conventional buffering agents such as weak acids/alkali metal salts of weak acids, e.g. acetic acid/sodium acetate; acid salts of polyprotic acids, e.g. sodium or potassium dihydrogen phosphate and disodium or dipotassium hydrogen phosphate; and the like.

A number of factors influence the choice of the aqueous acidic medium, particularly with regard to water content and pH. A greater water content in the medium is generally associated with increased reaction rate; however, it is generally also associated with lower solubility of the starting ketal and the rearranged esters in the medium. The rearrangement reaction produces a mole of hydrogen halide or a sulfonic acid per mole of ketal rearranged; thereby tending to lower the pH. A higher pH may be associated with increased racemization of isomeric acids and esters. Accordingly, balancing these factors, it may in some cases be desirable to control the pH and/or water content of the medium during the rearrangement step (a) by the addition of buffering agents and/or water during the reaction.

The aqueous acidic medium may also contain cosolvents for the ketals, i.e liquids, miscible with the medium, which enhance the solubility of the ketal in the medium. Such solvents may include polar protic liquids such as alcohols (e.g. isopropanol), diols (e.g. propylene glycol), ketones, amides, and the like. The cosolvents should preferably not be of such volatility that the boiling point of the medium is significantly depressed, as an elevated temperature is generally desirable for the rearrangement.

For the cyclic ketals, the reaction temperature is preferably between 60 °C and 200 °C, for example between 80 °C and the atmospheric pressure reflux temperature of the solution. A preferred temperature range is between 80 °C and 120 °C. For the acyclic ketals, the reaction temperature is preferably somewhat lower, e.g 25 °C-100 °C.

The reaction time depends on several factors, such as the reactivity of the starting ketal (I), the nature of the aqueous acidic medium (e.g. pH, solubility of the ketal in the medium), and the reaction temperature, and may vary between a few minutes to a few days. Typical reaction times are between 4 and 40 hours, such as between 10 and 20 hours.

Following the rearrangement step (a), the reaction mixture contains one or more compounds of formula (II),

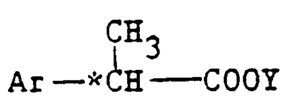

(II)

the nature of the mixture depending on the starting ketal and the reaction conditions. Where $R_1$ and $R_2$ in the starting ketal are alkyl, -Y will generally be $-R_1$ and $-R_2$; where $R_1$ and $R_2$ together are alkylene, -Y will

generally be $-R_1-R_2-OH$, $-R_2-R_1-OH$, the esters of those alcohols with the acids present in the aqueous acidic medium, and some quantities of $-R_1-R_2-X$ and $-R_2-R_1-X$ (where X is the halogen or the sulfonyloxy group of the ketal). For example, in the case of the bromo ethylene ketal rearranged in an acetic acid-containing medium, -Y may be $-CH_2CH_2OH$, $-CH_2CH_2OCOCH_3$, and possibly $-CH_2CH_2Br$. Typically, also, some hydrolysis of the rearranged esters will occur during the rearrangement step to produce some α-methylareneacetic acid; so that Y includes H. The compounds of formula (II) may either be isolated from the reaction mixture before hydrolysis step (b), or the reaction mixture as a whole may be subjected to hydrolysis step (b).

If it is desired to isolate the compounds of formula (II) from the reaction mixture formed in rearrangement step (a), this may be done by any of the techniques conventional in the art of such isolations; typically, by dilution of the reaction mixture and extraction of the α-methylareneacetic acid ester into a water-immiscible organic solvent, followed by evaporation of the solvent.

Hydrolysis step (b) may be performed by any of the techniques conventional in the art of ester hydrolysis, a.g. acidic or basic hydrolysis. Basic hydrolysis (e.g. the use of an aqueous alcoholic base) may be employed for the racemic α-methylareneacetic acids, but acidic hydrolysis is preferred for the isomeric acids. Thus, e.g., an aqueous mineral acid is added to the compounds of formula (II), either as such or in the reaction mixture from rearrangement step (a), and the resulting mixture is heated for a time sufficient to complete the hydrolysis of the compounds of formula (II) to the desired α-methylareneacetic acid of formula (III).

The α-methylareneacetic acid of formula (III) may be isolated from the reaction mixture of hydrolysis step (b) by any of the techniques conventional in the art of such isolations; typically, by dilution of the reaction mixture and extraction of the α-methylareneacetic acid into a water-immiscible organic solvent, followed by evaporation of the solvent.

The rearrangement step (a) and hydrolysis step (b) may be followed for the course of the reaction by conventional techniques such as HPLC, TLC, near-IR spectroscopy, and the like.

If another α-methylareneacetic acid is desired, rather than the direct acid product of rearrangement and hydrolysis of the starting ketal, the process also includes a conversion step (c), in which the compound of formula II formed in rearrangement step (a) is converted to the corresponding compound of formula II with the desired arene group or the acid formed in hydrolysis step (b) is converted to the desired acid.

Thus, for example, if a precursor to naproxen, such as 6-hydroxy-α-methyl-2-naphthaleneacetic acid or a 5-halo-6-methoxy-α-methyl-2-naphthaleneacetic acid, is prepared by the rearrangement/hydrolysis process of this invention, this precursor may be converted into naproxen by methods conventional in the art of such conversions. For example, the dehalogenation of 5-halo-6-methoxy- α-methyl-2-naphthaleneacetic acid and the methylation of 6-hydroxy-α-methyl-2-naphthaleneacetic acid are both described in European Published Application No. 0 110 671. Similar conversions may be applied at the ester stage, i.e. after rearrangement step (a).

If a mixture of isomers of ketal (I) has been employed as the starting material but a single isomer, such as the S-isomer, of the α-methylareneacetic acid is desired, rather than the mixture of isomers prepared in hydrolysis step (b), the process also includes a resolution step (d), in which the compound of formula II is resolved to yield the desired isomer or the acid formed in hydrolysis step (b) is resolved to yield the desired isomer.

Thus, for example, if (RS)-2-(-1-bromoethyl)-2-(6-methoxy-2-naphthyl)-5,5-dimethyl-1,3-dioxane is used as the starting ketal, so that the product of hydrolysis step (b) is (RS)-6-methoxy-α-methyl-2-naphthaleneacetic acid, the (S)-6-methoxy-α-methyl-2-naphthaleneacetic acid (naproxen) may be prepared by methods conventional in the art of such resolutions. Such techniques include fractional crystallization of diastereomeric salts formed with amines such as cinchonidine, N-alkyl-D-glucamines, and the like, or diastereomeric amides formed with amines such as α-methylbenzenemethanamine (α-phenethylamine), 2-amino-1-butanol, and the like.

If both conversion step (c) and resolution step (d) are desired to be performed, they may be performed in either order.

If a salt of the α-methylareneacetic acid is desired, rather than the acid itself, the process also includes a salt formation step (e), in which the acid formed in hydrolysis step (b), optionally after conversion and/or resolution, is neutralized to the desired salt.

This salt formation step (d) may be performed by any of the techniques conventional in the art of formation of salts of alkanoic acids, typically by dissolution of the α-methylareneacetic acid in an aqueous or aqueous alcoholic solution of the hydroxide of the desired cation, followed by precipitation of the salt by cooling and/or the addition of appropriate organic solvents. The resulting salt may be purified, if desired, by recrystallization or similar conventional techniques.

Thus, for example, sodium (S)-6-methoxy-α-methyl-2-naphthaleneacetate (naproxen sodium) is prepared by adding (S)-6-methoxy-α-methylnaphthaleneacetic acid to a solution of sodium hydroxide in aqueous methanol, and removal of the solvent to yield the desired salt.

## EXAMPLES

The following Examples illustrate the invention, but are not intended to limit its scope.

### Example 1

a) Ketal rearrangement: (RS)-2-(1-bromoethyl)-2-(6-methoxy-2-naphthyl)-5,5-dimethyl-1,3-dioxane (0.32 g) and sodium acetate (0.64 g) were dissolved in an aqueous solution of acetic acid (80%, 4 mL). The mixture was heated to 110 °C, and maintained at that temperature for 16 hours. After cooling to room temperature, the reaction mixture was poured into water (30 mL). The resulting solution was extracted with ethyl acetate (20 mL); and the ethyl acetate washed with water (30 mL) and aqueous sodium bicarbonate (30 mL), dried over anhydrous sodium sulfate, and evaporated to dryness under reduced pressure in a rotary evaporator, affording approximately 0.22 g of an oily product.

The crude product was chromatographed on silica gel using 10% ethyl acetate in hexane as solvent to yield 3-hydroxy-2,2-dimethylpropyl (RS)-6-methoxy-α-methyl-2-naphthaleneacetate (approximately 0.07-0.08 g) and 3-acetoxy-2,2-dimethylpropyl (RS)-6-methoxy-α-methyl-2-naphthaleneacetate (approximately 0.08-0.09 g).

(b) Hydrolysis: The crude reaction product from rearrangement step (a) was hydrolyzed with aqueous alcoholic base to afford (RS)-6-methoxy-α-methyl-2-naphthaleneacetic acid.

### Example 2

(a) Ketal rearrangement: (1′RS)-2-(1-bromoethyl)-2-(6-methoxy-2-naphthyl)-4,5-dimethyl-1,3-dioxolane (0.55 g), prepared from racemic 2,3-butanediol, potassium acetate (0.75 g), glacial acetic acid (4.5 mL) and water (0.5 mL) were heated to 125-130 °C in a stoopered 50 mL roundbottom flask, and maintained at that temperature with stirring for 36 hours. After cooling to room temperature, the reaction mixture was poured into water (30 mL). The resulting solution was extracted with ethyl acetate (20 mL); and the ethyl acetate washed with water (3 x 10 mL) and aqueous sodium bicarbonate (1 x 20 mL), dried over anhydrous sodium sulfate, and evaporated to dryness under reduced pressure in a rotary evaporator, affording approximately 0.3 g of an oily product.

The crude product was dissolved in a minimum volume of 20:80 dichloromethane/hexane and percolated over silica gel (5 g), eluted with 20:80 - 50:50 dichloromethane/hexane, and the solvent evaporated to yield 2-hydroxy-1-methylpropyl (RS)-6-methoxy-α-methyl-2-naphthaleneacetate and 2-acetoxy-1-methylpropyl (RS)-6-methoxy-α-methyl-2-naphthaleneacetate.

(b) Hydrolysis: The crude reaction product from rearrangement step (a) was hydrolyzed with aqueous alcoholic base to afford (RS)-6-methoxy-α-methyl-2-naphthaleneacetic acid.

### Example 3

(S)-2-(1-bromoethyl)-2-(6-methoxy-2-naphthyl)-5,5-dimethyl-1,3-dioxane (100 mg, approximately 95% e.e.) was dissolved in a 30% aqueous solution of propionic acid (1.3 mL) which had been buffered to pH 4.5-5 by the addition of 2.5 equivalents of sodium acetate. The mixture was heated to reflux temperature (oil bath at 110-115 °C), and maintained at that temperature for 16 hours. The reaction mixture was acidified with concentrated hydrochloric acid (6 drops), and heated at 90-100 °C overnight to effect complete hydrolysis of the rearranged ester product. The resulting solution was cooled to room temperature, diluted with water, and extracted with ethyl acetate (15 mL); and the ethyl acetate washed with water (3 x 15 mL).

The ethyl acetate layer was treated with aqueous sodium bicarbonate (20 mL), and the ethyl acetate layer then discarded. The basic aqueous layer was reacidified with dilute hydrochloric acid and extracted with ethyl acetate. The ethyl acetate layer was washed with water, dried over sodium sulfate, and evaporated to dryness to afford (S)-6-methoxy-α-methyl-2-naphthaleneacetic acid (0.045 g, 75% yield, approximately 93% e.e.).

Example 4

(S)-2-(1-bromoethyl)-2-(6-methoxy-2-naphthyl)-5,5-dimethyl-1,3-dioxane (100 mg, approximately 95% e.e.) was dissolved in a 30% aqueous solution of propionic acid (1.3 mL) which had been buffered to pH 2.5-3 by the addition of dipotassium hydrogen phosphate. The mixture was heated to reflux temperature (oil bath at 110-115° C), and maintained at that temperature for 7 hours. The reaction mixture was acidified with concentrated hydrochloric acid (4 drops) to reduce the pH below 2, and heated at 90-110° C overnight to effect complete hydrolysis of the rearranged ester product. The resulting solution was cooled to room temperature, diluted with water, and extracted with ethyl acetate (15 mL); and the ethyl acetate washed with water (3 x 15 mL). The ethyl acetate layer was treated with aqueous sodium bicarbonate (30 mL), and the ethyl acetate layer then discarded. The basic aqueous layer was reacidified with dilute hydrochloric acid and extracted with ethyl acetate. The ethyl acetate layer was washed with water, dried over sodium sulfate, and evaporated to dryness to afford (S)-6-methoxy-α-methyl-2-naphthaleneacetic acid (0.036 g, 60% yield, approximately 92% e.e.).

Example 5

0.5 g (1) (S)-2-(-1-methanesulfonyloxyethyl)-2-(6-methoxy-2-naphthyl)-5,5-dimethyl-1,3-dioxane dissolved in 5 ml of a 50/50 propionic acid/$H_2O$ mixture containing 1.5 equivalents of potassium acetate was heated at 80° C for about 8 hours. The reaction mixture was acidified with about 1 ml of 5N HCl and heated at 100° C overnight to afford complete hydrolysis of the rearranged ester. The resulting solution was cooled, filtered, and washed with $H_2O$. The solid cake was redissolved in ethyl acetate and treated with $NaHCO_3$ solution. The aqueous layer was separated, reacidified with 2N HCl and extracted with ethyl acetate. The ethyl acetate solution was evaporated under vacuum affording a white solid identified as pure (S)-2-6-methoxy-α-methyl-2-naphthaleneacetic acid.

Example 6

0.4 g of (S)-2-(1-methanesulfonyloxyethyl)-2-(6-methoxy-2-naphthyl)-1,3-dioxolane dissolved in 4 ml of a 50/50 propionic acid/$H_2O$ mixture containing 2-3 equivalents of potassium acetate was heated at 100° C for 24 hours. The reaction mixture was acidified with 1-2 ml of 5N HCl and heated at 60° C for 16-24 hours to afford complete hydrolysis of the rearranged ester. The resulting solution was cooled, filtered, and washed with $H_2O$. The solid cake was redissolved in ethyl acetate and treated with $NaHCO_3$ solution. The aqueous layer was separated, reacidified with 2N HCl and extracted with ethyl acetate. The ethyl acetate solution was evaporated under vacuum affording a white solid identified as pure (S)-2-6-methoxy-α-methyl-2-naphthaleneacetic acid (>97% e.e.).

## Claims

1. A process for the preparation of an α-methylareneacetic acid, which comprises
(a) treating a ketal of a compound of the formula

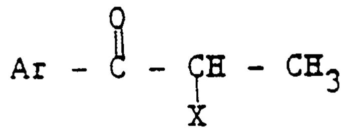

wherein Ar is an aryl group; X is halogen or a group of the formula

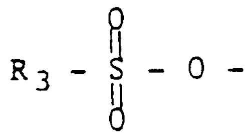

wherein $R_3$ is alkyl, cycloalkyl, alkenyl, alkynyl, aryl or arylalkyl, in an aqueous acidic medium to cause rearrangement of the ketal to an ester of the corresponding α-methylareneacetic acid; and

(b) hydrolyzing the thus-formed ester to yield the α-methylareneacetic acid, with the proviso that if the ketal is a ketal of a 1-aryl-2-halo-1-propanone and is cyclic, it contains not more than one carboxy group.

2. A process of Claim 1 wherein the ketal is of the formula

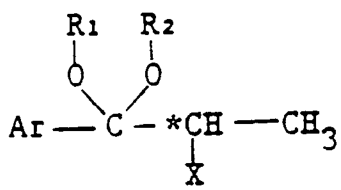

(I)

in which
Ar is aryl;
each of $R_1$ and $R_2$ is individually alkyl, or $R_1$ and $R_2$ together are alkylene; and
X is halogen or a group of the formula

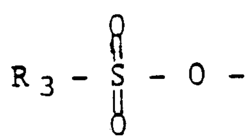

wherein $R_3$ is alkyl, cycloalkyl, alkenyl, alkynyl, aryl or arylalkyl.

3. A process of Claim 1 or Claim 2 wherein the ketal is optically active.

4. A process of Claim 1 or Claim 2 wherein the ketal is racemic.

5. A process of any one of any of the preceding claims wherein the rearrangement step (a) is carried out at a pH between 1 and 6.

6. A process of any one of the preceding claims wherein the rearrangement step (a) is carried out in an aqueous solution of a lower alkanoic acid.

7. A process of Claim 6 wherein the aqueous solution of a lower alkanoic acid also contains a buffer.

8. A process of any one of the preceding claims wherein the rearrangement step (a) is carried out in the presence of a cosolvent for the ketal.

9. A process of any one of the preceding Claims wherein the rearrangement step (a) is carried out at a temperature between 80 °C and 200 °C.

10. A process of any one of the preceding claims wherein the hydrolyzing step (b) is carried out by acidic catalysis.

11. A process for the preparation of (RS)-6-methoxy-α-methyl-2-naphthaleneacetic acid, which comprises:

(a) treating a ketal of the formula

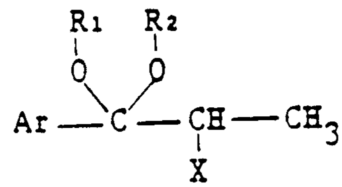

in which
Ar is 6-methoxy-2-naphthyl, 5-chloro-6-methoxy-2-naphthyl 5-bromo-6-methoxy-2-naphthyl, 6-hydroxy-2-naphthyl, or 5-bromo-6-hydroxy-2-naphthyl;
each of $R_1$ and $R_2$ is individually alkyl, or $R_1$ and $R_2$ together are alkylene; and
X is halogen or a group of the formula

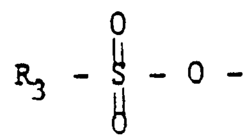

wherein $R_3$ is alkyl, cycloalkyl, alkenyl, alkynyl, aryl or arylalkyl;
in an aqueous acidic medium to cause rearrangement of the ketal to an ester of an acid of the formula (II)

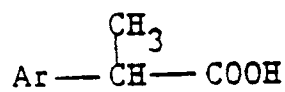

, ;

and

(b) hydrolyzing the thus-formed ester to yield the corresponding acid; and,

if Ar is not 6-methoxy-2-naphthyl,

(c) converting the compound formed in rearrangement step (a) to the corresponding compound in which Ar is 6-methoxy-2-naphthyl, or converting the acid formed in hydrolysis step (b) to (RS)-6-methoxy-α-methyl-2-naphthaleneacetic acid.

12. A process for the preparation of (S)-6-methoxy-α-methyl-2-naphthaleneacetic acid, which comprises:

(a) treating a ketal of the formula

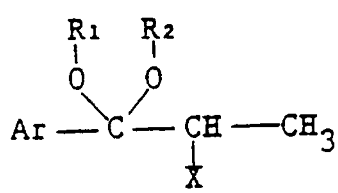

in which
Ar is 6-methoxy-2-naphthyl, 5-chloro-6-methoxy-2-naphthyl, 5-bromo-6-methoxy-2-naphthyl, 6-hydroxy-2-naphthyl, or 5-bromo-6-hydroxy-2-naphthyl;
each of $R_1$ and $R_2$ is individually alkyl, or $R_1$ and $R_2$ together are alkylene; and
X is halogen or a group of the formula

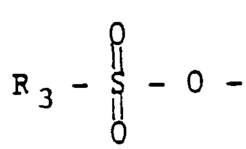

wherein $R_3$ is alkyl, cycloalkyl, alkenyl, alkynyl, aryl or arylalkyl;
in an aqueous acidic medium to cause rearrangement of the ketal to an ester of an acid of the formula

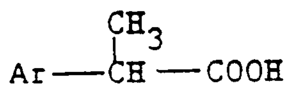

;

and

(b) hydrolyzing the thus-formed ester to yield the corresponding acid; and,

if Ar is not 6-methoxy-2-naphthyl,

(c) converting the compound formed in rearrangement step (a) to the corresponding compound in which Ar is 6-methoxy-2-naphthyl, or converting the acid formed in hydrolysis step (b) to (S)-6-methoxy-α-methyl-2-naphthaleneacetic acid.

13. A process of Claim 11 or Claim 12 wherein Ar is 6-methoxy-2-naphthyl.

14. A process of Claims 11, 12 or 13 wherein X is Br, methanesulfonyloxy, benzenesulfonyloxy, or toluenesulfonyloxy.

15. A process of any one of claims 11 to 14 wherein $R_1$ and $R_2$ together are $-CH(CH_3)-CH(CH_3)-$, $--(CH_2)_3-$, or $-CH_2-C(CH_3)_2-CH_2-$.

16. A process for the preparation of (5)-6-methoxy-α-methyl-2-naphthaleneacetic acid, which comprises preparing (RS)-6-methoxy-α-methyl-2-naphthaleneacetic acid by the process of Claim 11 followed by

(d) resolving the thus-formed (RS)-6-methoxy-α-methyl-2-naphthaleneacetic acid.

17. A process for the preparation of sodium (5)-6-methoxy-α-methyl-2-naphthaleneacetate, which comprises preparing (S)-6-methoxy-α-methyl-2-naphthaleneacetic acid by the process of Claim 12 followed by

(d) neutralizing the thus-formed (S)-6-methoxy-α-methyl-2-naphthaleneacetic acid with an aqueous alcoholic solution of sodium hydroxide.